# EUROPEAN PATENT APPLICATION

(11) **EP 4 748 329 A1**
(43) Date of publication of application: **27.05.2026**
(21) Application number: 25214977.8
(22) Date of filing: 11.11.2025
(51) Int. Cl.: A61B 18/12, A61B 18/14, A61N 1/32, A61N 1/04, A61N 1/05, A61N 1/06

(54) **SKIN BEAUTIFYING DEVICE**

(30) Priority: 26.11.2024 KR 20240171008
(71) Applicant: IDS Ltd Co., Ltd., Paju-si, Gyeonggi-do (KR); A.M.I Inc., Seoul (KR)
(72) Inventor: AHN, Kwang Yeong, Seoul (KR)
(74) Representative: Danubia Patent & Law Office LLC

(57) **Abstract**

According to the present disclosure, it is possible to precisely adjust a degree of insertion of the plurality of needles fixed to the needle bundle in consideration of skin thickness (for example, a thickness from epidermis to dermis of the skin) to an extent that a sufficient skin beautification effect is expected, and when an error occurs in the degree of insertion of the needles due to forward or rearward movement of the conveyor during use, the error in the degree of insertion of the needles can be easily corrected by correcting the starting point position of the conveyor using a starting point position detector.

## Description

### CROSS-REFERENCE TO RELATED APPLICATION

This application claims priority to and the benefit of Korean Patent Application No. 10-2024-0171008, filed on NOV 26, 2024, the disclosure of which is incorporated herein by reference in its entirety.

### BACKGROUND

### 1. Field of the Invention

The present disclosure relates to a skin beautifying device, and more particularly, to a skin beautifying device that promotes skin regeneration by conducting alternating current (AC) (for example, high-frequency current, etc.) into the skin through a plurality of needles.

### 2. Discussion of Related Art

A fractional device for skin beautification that is disclosed in Patent Document 1 is inserted into the skin, conducts alternating current (AC) (for example, high-frequency current, etc.) into the skin through a plurality of needles, which serve as electrodes that receive electrical energy, and applies local thermal damage to the skin, thereby forming fine wounds for promoting skin regeneration from epidermis to dermis of the skin.

It is known that when AC (for example, high-frequency current, etc.) is conducted into the skin through a plurality of needles and fine wounds are formed as described above, new collagen formation in the dermis layer of the skin can be promoted, elastic fibers can be regenerated, and accordingly, it is possible to treat skin diseases such as skin wrinkles, enlarged pores, acne scars, stretch marks, traumatic scars, surgical scars, and burn scars.

Meanwhile, the fractional device for skin beautification that is disclosed in Patent Document 1 has a structure in which a needle panel to which the plurality of needles are fixed is immovable, and since only portions of the plurality of needles that protrude a predetermined length from the needle panel come into contact with the skin or are inserted into the dermis when an external force is applied to a handpiece connected to a main body, there is a disadvantage in that it is difficult to precisely adjust a degree of insertion of the needles in consideration of skin thickness (for example, a thickness from epidermis to dermis of the skin) to an extent that a sufficient skin beautification effect is expected.

### [Related Art Document]

### [Patent Document]

(Patent Document 1) KR10-0946363 B1

### SUMMARY OF THE INVENTION

The present disclosure is directed to providing a skin beautifying device in which, as a conveyor that is screw-coupled to a ball screw rotating shaft of a step motor, moves forward from a starting point position, and then moves rearward and returns to the starting point position moves forward a moving distance corresponding to a forward movement signal of the step motor and pushes a needle bundle, a plurality of needles fixed to the needle bundle are precisely inserted into skin to an extent corresponding to the forward movement signal, alternating current (AC) (for example, high-frequency current, etc.) supplied via the conveyor is conducted into the skin through the plurality of needles for a predetermined amount of time to form fine wounds for promoting regeneration of the skin, and when it is detected that the conveyor has moved rearward a moving distance corresponding to a rearward movement signal of the step motor and has returned to the starting point position, the conveyor is moved forward again a moving distance corresponding to a starting point correction driving signal of the step motor to correct the starting point position.

According to one aspect of the present disclosure, there is provided a skin beautifying device that includes a main body and a handpiece connected to the main body via a wire and forms fine wounds for promoting regeneration of skin by a plurality of needles installed in the handpiece being inserted into the skin, conducting alternating current (AC) for a predetermined amount of time, and applying local thermal damage, wherein the main body is configured to include a controller that adjusts an AC application time during which AC is applied to the plurality of needles, that repeatedly performs, at predetermined time intervals, a process of outputting a motor driving signal for adjusting a degree of insertion of the plurality of needles into the skin while a motor driving request is present, supplying the AC to the plurality of needles inserted into the skin during the AC application time, and then taking the plurality of needles out of the skin, and that stops outputting the motor driving signal in response to a stop of the motor driving request, a power adjuster that adjusts a voltage level of direct current (DC) power that is supplied to generate AC supplied to the handpiece, and an AC generator that converts the DC power supplied from the power adjuster into AC power to generate the AC supplied to the handpiece, and the handpiece is configured to include a handpiece housing that is hollow and serves as a handle, a needle bundle cover that is hollow, is coupled to or separated from an end of the handpiece housing, and has a plurality of needle protrusion holes, which correspond one-to-one to the plurality of needles, formed in a front surface and an opening formed at a rear surface, a step motor driver that is accommodated in the handpiece housing, that repeatedly performs, at predetermined time intervals, a skin beautifying process of transmitting the motor driving request input by manipulation of a button to the controller, outputting the forward movement signal upon receiving the motor driving signal, and then outputting the rearward movement signal after an elapse of the AC application time, and that stops transmitting the motor driving request to the controller in response to a stop of an input of the motor driving request, a step motor that is accommodated in the handpiece housing and has a ball screw rotating shaft that performs a predetermined number of forward rotations corresponding to the forward movement signal or performs a predetermined number of rearward rotations corresponding to the rearward movement signal, a needle bundle that is accommodated in the needle bundle cover and in which a positive electrode thin film conductive layer and a negative electrode thin film conductive layer are stacked on a front surface and a rear surface, respectively, of a central insulating plate, a surface of the positive electrode thin film conductive layer and a surface of the negative electrode thin film conductive layer pass through a front surface and a rear surface, respectively, of a needle bundle plate covered with an insulating layer, the plurality of needles, which correspond one-to-one to a plurality of through-holes arranged in a lattice shape, are fixed by soldering while inserted into the plurality of through-holes, during supply of the AC, the plurality of needles alternately have a positive polarity and a negative polarity while neighboring each other in a horizontal direction and a vertical direction of the lattice shape, all of the positive polarity needles among the plurality of needles are connected to be electrically connected to the positive electrode thin film conductive layer formed on the front surface, all of the negative polarity needles among the plurality of needles are connected to be electrically connected to the negative electrode thin film conductive layer formed on the rear surface, and a positive electrode terminal part connected to the positive electrode thin film conductive layer and a negative electrode terminal part connected to the negative electrode thin film conductive layer are formed on a left side edge and a right side edge, respectively, of the front surface, an AC terminal plate that is accommodated in the needle bundle cover and has a front surface adhered and coupled to a rear surface of the needle bundle and a pair of AC supply terminal parts, which are spaced apart from each other and connected to be electrically connected to the positive electrode terminal part and the negative electrode terminal part of the needle bundle, disposed on a surface of a central portion of a rear surface, and a conveyor that is accommodated in the handpiece housing, has a pair of terminal pins, which come into contact with the pair of AC supply terminal parts of the AC terminal plate and supply the AC to the plurality of needles of the needle bundle, protruding from a front end protruding part, is connected to be electrically connected to the pair of terminal pins through wires inserted into wire connection holes formed in a pair of protruding parts of left and right side surface parts, applies the AC supplied from the AC generator to the pair of terminal pins, is screw-coupled to the ball screw rotating shaft of the step motor through a screw hole in a rear surface part, moves forward from a starting point position and then moves rearward and returns to the starting point position, moves forward a moving distance corresponding to a forward movement signal of the step motor and pushes the needle bundle so that the plurality of needles fixed to the needle bundle are precisely inserted into the skin to an extent corresponding to the forward movement signal, supplies the AC to the plurality of needles of the needle bundle via the pair of terminal pins, the pair of AC supply terminal parts of the AC terminal plate, and the positive electrode terminal part and the negative electrode terminal part of the needle bundle so that the AC is conducted into the skin through the plurality of needles for a predetermined amount of time and local thermal damage is applied to the skin to form fine wounds for promoting regeneration of the skin, and moves rearward a moving distance corresponding to a rearward movement signal of the step motor and returns to the starting point position.

The skin beautifying device according to the present disclosure may further include a starting point position detector that is accommodated in the handpiece housing and outputs a starting point position detection signal and transmits the starting point position detection signal to the controller upon detecting that the conveyor has moved rearward the moving distance corresponding to the rearward movement signal of the step motor and a position detection protrusion protruding from a rear side portion of the conveyor has returned to the starting point position, and when a starting point correction request is input by manipulation of a button (not illustrated), the controller may output a starting point correction driving signal to the step motor driver so that the step motor driver outputs a starting point correction signal, and when deviation of the position detection protrusion of the conveyor from the starting point position is detected by the starting point position detector while the step motor rotates the ball screw rotating shaft forward a predetermined number of forward rotations corresponding to the starting point correction signal, the controller may stop outputting the starting point correction driving signal to the step motor driver and may correct the current position of the conveyor to the starting point position.

The skin beautifying device according to the present disclosure may further include a needle bundle holder that is accommodated in the needle bundle cover, allows the AC terminal plate to be fixed as an edge portion of the needle bundle, which is adhered and coupled to a rear surface, is caught at an opening in a front surface of a body having a shape that is open in a front-rear direction, has a terminal pin hole, through which the pair of terminal pins of the conveyor pass and come into contact with the pair of AC supply terminal parts of the AC terminal plate, formed in a central portion of a rear surface cover that covers an opening in a rear surface of the body, has a plurality of protrusions formed along an edge of the rear surface cover, and allows the needle bundle to move forward when a front end of the conveyor moves forward and pushes the rear surface cover while in contact with the rear surface cover.

The skin beautifying device according to the present disclosure may further include: a needle bundle detection plate that is accommodated in the handpiece housing, has a central through-hole formed therein through which the front end protruding part of the conveyor passes, has a front surface at which the pair of protruding parts of the left and right side surface parts of the conveyor are caught and fixed, has a pair of needle bundle detection parts, which are spaced apart from each other with the central through-hole disposed therebetween and include at least one or more detection pins, disposed side by side on the front surface, and has a needle bundle detection switch disposed at a proper place on the front surface that is spaced apart from the pair of needle bundle detection parts; and a needle bundle cover plate that is accommodated in the needle bundle cover, has a central through-hole formed therein through which the front end protruding part of the conveyor passes, has a pair of needle bundle detection contact point parts, which are spaced apart from each other with the central through-hole disposed therebetween and include at least one or more detection contact points, disposed side by side on a rear surface facing the needle bundle detection plate, and has a magnetic part, which turns on the needle bundle detection switch due to a magnetic force when the pair of needle bundle detection parts of the needle bundle detection plate and the pair of needle bundle detection contact point parts electrically come into contact as the needle bundle cover is fixed while locked to an end cover of the handpiece housing, and a memory, which records a number of contacts every time the pair of needle bundle detection parts and the pair of needle bundle detection contact point parts electrically come into contact, disposed at proper places on the rear surface.

In the skin beautifying device according to the present disclosure, the controller may generate an alert for stopping use of the needle bundle when the number of contacts recorded in the memory of the needle bundle cover plate that is accommodated in the needle bundle cover exceeds a predetermined value or more.

In the skin beautifying device according to the present disclosure, the step motor driver may transmit the motor driving request input by manipulation of a button to the controller in a state in which the needle bundle detection switch of the needle bundle detection plate accommodated in the handpiece housing is turned on.

In the skin beautifying device according to the present disclosure, a plurality of movement guide grooves, into which a spring is inserted, into which the plurality of protrusions formed along the edge of the rear surface cover of the needle bundle holder are inserted with one-to-one correspondence, and which guides forward movement while pressing the spring during forward movement of the needle bundle, may be formed in an inner side surface of the opening in the rear surface of the needle bundle cover, and during rearward movement of the conveyor, a restoring force of the spring may cause the plurality of needles protruding through the plurality of needle protrusion holes of the needle bundle cover to return to an inner portion of the needle bundle cover.

In the skin beautifying device according to the present disclosure, a pair of locking protrusions facing each other may be formed on an inner side surface close to an edge of the opening in the rear surface of the needle bundle cover, the pair of locking protrusions may rotate after being inserted into an L-shaped locking groove formed in an outer circumferential surface of a tip of the end cover of the handpiece housing and may fix the needle bundle cover in a state in which the needle bundle cover is locked to the end cover of the handpiece housing, and a catching groove that prevents the locked state from being released may be formed in the L-shaped locking groove.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a view illustrating a configuration of a skin beautifying device according to an embodiment of the present disclosure;
FIG. 2 is a perspective view illustrating a handpiece of FIG. 1;
FIG. 3 is a front exploded view of FIG. 2;
FIG. 4 is a rear exploded view of FIG. 2;
FIG. 5 is a cross-sectional view of a needle bundle plate;
FIG. 6 is a plan view illustrating a positive electrode thin film conductive layer of the needle bundle plate;
FIG. 7 is a plan view illustrating a negative electrode thin film conductive layer of the needle bundle plate;
FIG. 8 is a plan view illustrating a coupling state of a step motor and a conveyor;
FIG. 9a is a plan view for describing forward movement operations of the conveyor; and
FIG. 9b is a plan view for describing rearward movement operations of the conveyor; and
FIG. 9c describes that the position detection protrusion of the conveyor deviates from the detection line, and
FIG. 10 is a front view of a needle bundle detection plate.

### DETAILED DESCRIPTION OF EXEMPLARY EMBODIMENTS

Hereinafter, embodiments of the present disclosure will be described in more detail with reference to the accompanying drawings.

A skin beautifying device according to the present disclosure, which will be described below, is not limited to the following embodiments, modifications may be made by those of ordinary skill in the art without departing from the gist of the technology claimed in the claims, and such modifications also fall within the technical spirit of the present disclosure.

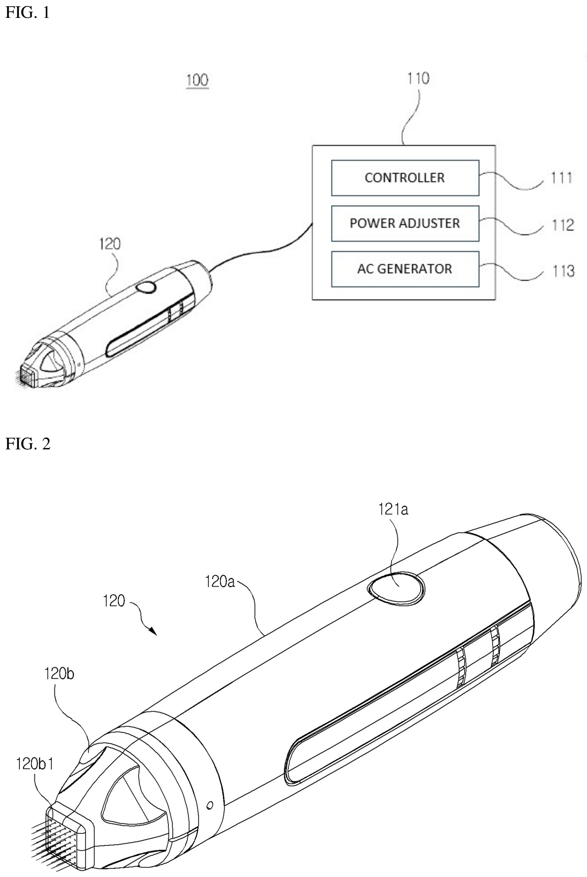

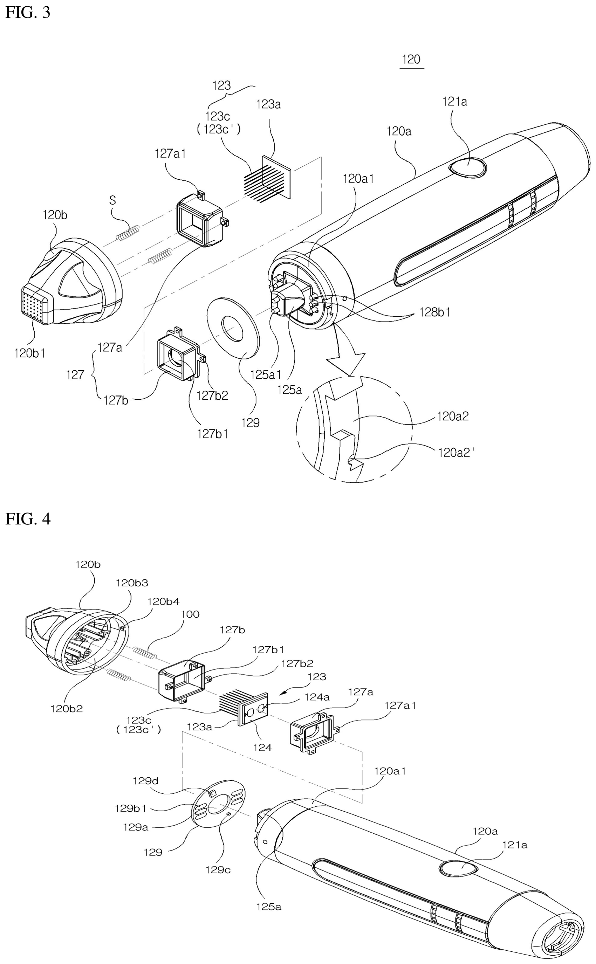

As shown in FIGS. 1 to 10, a skin beautifying device 100 according to the present disclosure includes a main body 110 and a handpiece 120 connected to the main body 110 via a wire, and fine wounds for promoting regeneration of skin are formed by a plurality of needles 123c and 123c' installed in the handpiece 120 being inserted into the skin, conducting alternating current (AC) for a predetermined amount of time, and applying local thermal damage.

The main body 110 is configured to include a controller 111, a power adjuster 112, and an AC generator 113.

The controller 111 adjusts an AC application time during which AC is applied to the plurality of needles 123c and 123c', repeatedly performs, at predetermined time intervals, a process of outputting a motor driving signal for adjusting a degree of insertion of the plurality of needles 123c and 123c' into the skin while a motor driving request is present, supplying the AC to the plurality of needles 123c and 123c' inserted into the skin during the AC application time, and then taking the plurality of needles 123c and 123c' out of the skin, and stops outputting the motor driving signal in response to a stop of the motor driving request.

The power adjuster 112 adjusts a voltage level of direct current (DC) power that is supplied to generate AC supplied to the handpiece 120, and for example, the power adjuster 112 may adjust the voltage level in 0.004 V units in a range of 8 V to 36 V of DC.

The AC generator 113 converts the DC power supplied from the power adjuster 112 into AC power to generate the AC supplied to the handpiece 120, and for example, the AC generator 113 may generate high-frequency current of 200 mA at 1 to 2 MHz.

The handpiece 120 is configured to include a handpiece housing 120a and a needle bundle cover 120b, a step motor driver 121, a step motor 122, a conveyor 125, a starting point position detector 126, and a needle bundle detection plate 128 are accommodated in the handpiece housing 120a, and a needle bundle 123, an AC terminal plate 124, a needle bundle holder 127, and a needle bundle cover plate 129 are accommodated in the needle bundle cover 120b.

Among the terms used below in giving description with reference to FIGS. 1 to 10, the expression "front surface" may be understood as an upper surface, one side surface, or a left side surface, and the expression "rear surface" may be understood as a lower surface, the other side surface, or a right side surface.

For example, in the case of the needle bundle cover 120b shown in FIGS. 2, 3, and 4, needle protrusion holes 120b1 formed in a front surface and an opening 120b2 formed at a rear surface may be understood as the needle protrusion holes 120b1 formed in one side surface or a left side surface and the opening 120b2 formed at the other side surface or a right side surface when viewed in a horizontal direction, and may be understood as the needle protrusion holes 120b1 formed in an upper surface and the opening 120b2 formed at a lower surface when viewed in a vertical direction.

Likewise, in the case of the needle bundle 123, the AC terminal plate 124, the needle bundle holder 127, and the needle bundle cover plate 129 accommodated in the needle bundle cover 120b, when viewed based on the needle bundle cover 120b in the horizontal direction, a side toward the step motor 122 may be understood as a front surface (or an upper surface or one side surface or a left side surface), and a side toward the needle bundle cover 120b may be understood as a rear surface (or a lower surface or the other side surface or a right side surface.

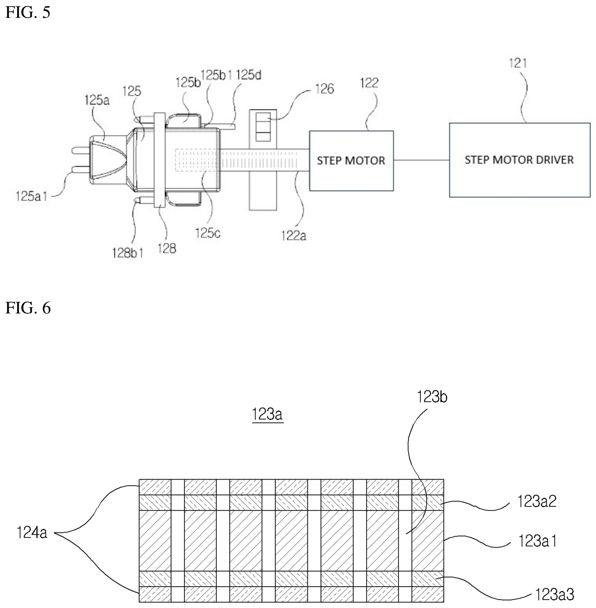

In addition, in the case of the conveyor 125 and the needle bundle detection plate 128 accommodated in the handpiece 120 and shown in FIGS. 5 to 10, when viewed based on the needle bundle cover 120b in the horizontal direction, a side toward the step motor 122 may be understood as a front surface (or an upper surface or one side surface or a left side surface), and a side toward the needle bundle cover 120b may be understood as a rear surface (or a lower surface or the other side surface or a right side surface.

The handpiece housing 120a is manufactured to be hollow and serves as a handle.

The needle bundle cover 120b is manufactured to be hollow, is coupled to or separated from an end of the handpiece housing 120a, and has a plurality of needle protrusion holes 120b1, which correspond one-to-one to the plurality of needles 123c and 123c', formed in a front surface and an opening 120b2 formed at a rear surface.

The step motor driver 121 is accommodated in the handpiece housing 120a, repeatedly performs, at predetermined time intervals, a skin beautifying process of transmitting a motor driving request input by manipulation of a button 121a to the controller 111, outputting a forward movement signal upon receiving the motor driving signal, and then outputting a rearward movement signal after an elapse of the AC application time, and stops transmitting the motor driving request to the controller 111 in response to a stop of an input of the motor driving request.

The step motor 122 is accommodated in the handpiece housing 120a and has a ball screw rotating shaft 122a that performs a predetermined number of forward rotations corresponding to the forward movement signal or performs a predetermined number of rearward rotations corresponding to the rearward movement signal.

In the needle bundle 123 that is accommodated in the needle bundle cover 120b, a positive electrode thin film conductive layer 123a2 and a negative electrode thin film conductive layer 123a3 are stacked on a front surface and a rear surface, respectively, of a central insulating plate 123a1, a surface of the positive electrode thin film conductive layer 123a2 and a surface of the negative electrode thin film conductive layer 123a3 pass through a front surface and a rear surface, respectively, of a needle bundle plate 123a covered with an insulating layer 123a4, and the plurality of needles 123c and 123c', which correspond one-to-one to a plurality of through-holes 123b arranged in a lattice shape, are fixed by soldering while inserted into the plurality of through-holes 123b.

During supply of the AC, the plurality of needles 123c and 123c' fixed to the needle bundle 123 alternately have a positive polarity and a negative polarity while neighboring each other in a horizontal direction and a vertical direction of the lattice shape.

All of the positive polarity needles 123c among the plurality of needles 123c and 123c' are connected to be electrically connected to the positive electrode thin film conductive layer 123a2 formed on the front surface, and all of the negative polarity needles 123c' among the plurality of needles 123c and 123c' are connected to be electrically connected to the negative electrode thin film conductive layer 123a3 formed on the rear surface.

A positive electrode terminal part 123a2' connected to the positive electrode thin film conductive layer 123a2 and a negative electrode terminal part 123a3' connected to the negative electrode thin film conductive layer 123a3 are formed on a left side edge and a right side edge, respectively, of the front surface of the needle bundle 123, preferably, on a left side edge and a right side edge, respectively, of a front surface of the needle bundle plate 123a.

The AC terminal plate 124 is accommodated in the needle bundle cover 120b and has a front surface adhered and coupled to a rear surface of the needle bundle 123 and a pair of AC supply terminal parts 124a, which are spaced apart from each other and connected to be electrically connected to the positive electrode terminal part 123a2' and the negative electrode terminal part 123a3' of the needle bundle 123, disposed on a surface of a central portion of a rear surface.

The conveyor 125 is accommodated in the handpiece housing 120a, has a pair of terminal pins 125a1, which come into contact with the pair of AC supply terminal parts 124a of the AC terminal plate 124 and supply the AC to the plurality of needles 123c and 123c' of the needle bundle 123, protruding from a front end protruding part 125a, and for example, the pair of terminal pins 125a1 may be formed as a pair of Pogo pins.

The conveyor 125 is connected to be electrically connected to the pair of terminal pins 125a1 through wires inserted into wire connection holes 125b1 formed in a pair of protruding parts 125b of left and right side surface parts and applies the AC supplied from the AC generator 113 to the pair of terminal pins 125a1.

The conveyor 125 is screw-coupled to the ball screw rotating shaft 122a of the step motor 122 through a screw hole 125c in a rear surface part, and moves forward from a starting point position and then moves rearward and returns to the starting point position.

The conveyor 125 moves forward a moving distance corresponding to a forward movement signal of the step motor 122 and pushes the needle bundle 123 so that the plurality of needles 123c and 123c' fixed to the needle bundle 123 are precisely inserted into the skin to an extent corresponding to the forward movement signal, supplies the AC to the plurality of needles 123c and 123c' of the needle bundle 123 via the pair of terminal pins 125a1, the pair of AC supply terminal parts 124a of the AC terminal plate 124, and the positive electrode terminal part 123a2' and the negative electrode terminal part 123a3' of the needle bundle 123 so that the AC is conducted into the skin through the plurality of needles 123c and 123c' for a predetermined amount of time and local thermal damage is applied to the skin to form fine wounds for promoting regeneration of the skin, and moves rearward a moving distance corresponding to a rearward movement signal of the step motor 122 and returns to the starting point position.

The starting point position detector 126 is accommodated in the handpiece housing 120a and outputs a starting point position detection signal and transmits the starting point position detection signal to the controller 111 upon detecting that the conveyor 125 has moved rearward the moving distance corresponding to the rearward movement signal of the step motor 122 and a position detection protrusion 125d protruding from a rear side portion of the conveyor 125 has returned to the starting point position.

Preferably, the starting point position detector 126 may be configured as a photocoupler.

When a starting point correction request is input by manipulation of a button (not illustrated), the controller 111 that has received the starting point position detection signal of the starting point position detector 126 outputs a starting point correction driving signal to the step motor driver 121 so that the step motor driver 121 outputs a starting point correction signal, and when deviation of the position detection protrusion 125d of the conveyor 125 from the starting point position is detected by the starting point position detector 126 while the step motor 122 rotates the ball screw rotating shaft 122a forward a predetermined number of forward rotations corresponding to the starting point correction signal, the controller 111 stops outputting the starting point correction driving signal to the step motor driver 121 and corrects the current position of the conveyor 125 to the starting point position.

The needle bundle holder 127 is accommodated in the needle bundle cover 120b and allows the AC terminal plate 124 to be fixed as an edge portion of the needle bundle 123, which is adhered and coupled to a rear surface, is caught at an opening in a front surface of a body 127a having a shape that is open in a front-rear direction.

The needle bundle holder 127 has a terminal pin hole 127b1, through which the pair of terminal pins 125a1 of the conveyor 125 pass and come into contact with the pair of AC supply terminal parts 124a of the AC terminal plate 124, formed in a central portion of a rear surface cover 127b that covers an opening in a rear surface of the body 127a.

The needle bundle holder 127 has a plurality of protrusions 127b2 formed along an edge of the rear surface cover 127b and allows the needle bundle 123 to move forward when a front end of the conveyor 125 moves forward and pushes the rear surface cover 127b while in contact with the rear surface cover 127b.

The plurality of protrusions 127b2 formed along the edge of the rear surface cover 127b are coupled while corresponding one-to-one to a plurality of protrusions 127a1 formed along an edge of the body 127a.

The needle bundle detection plate 128 is accommodated in the handpiece housing 120a, has a central through-hole 128a formed therein through which the front end protruding part 125a of the conveyor 125 passes, has a front surface at which the pair of protruding parts 125b of the left and right side surface parts of the conveyor 125 are caught and fixed, has a pair of needle bundle detection parts 128b, which are spaced apart from each other with the central through-hole 128a disposed therebetween and include at least one or more detection pins 128b1, disposed side by side on the front surface, and has a needle bundle detection switch 128c disposed at a proper place on the front surface that is spaced apart from the pair of needle bundle detection parts 128b.

The at least one or more detection pins 128b1 may be formed as at least one or more Pogo pins.

The needle bundle cover plate 129 is accommodated in the needle bundle cover 120b, has a central through-hole 129a formed therein through which the front end protruding part 125a of the conveyor 125 passes, and has a pair of needle bundle detection contact point parts 129b, which are spaced apart from each other with the central through-hole 129a disposed therebetween and include at least one or more detection contact points 129b1, disposed side by side on a rear surface facing the needle bundle detection plate 128.

The needle bundle cover plate 129 has a magnetic part 129c, which turns on the needle bundle detection switch 128c due to a magnetic force when the pair of needle bundle detection parts 128b of the needle bundle detection plate 128 and the pair of needle bundle detection contact point parts 129b electrically come into contact as the needle bundle cover 120b is fixed while locked to an end cover 120a1 of the handpiece housing 120a, and a memory 129d, which records a number of contacts every time the pair of needle bundle detection parts 128b and the pair of needle bundle detection contact point parts 129b electrically come into contact, disposed at proper places on the rear surface.

The controller 111 generate an alert for stopping use of the needle bundle 123 when the number of contacts recorded in the memory 129d of the needle bundle cover plate 129 that is accommodated in the needle bundle cover 120b exceeds a predetermined value or more.

The step motor driver 121 transmits the motor driving request input by manipulation of the button 121a to the controller 111 in a state in which the needle bundle detection switch 128c of the needle bundle detection plate 128 accommodated in the handpiece housing 120a is turned on.

A plurality of movement guide grooves 120b3, into which a spring S is inserted, into which the plurality of protrusions 127b2 formed along the edge of the rear surface cover 127b of the needle bundle holder 127 are inserted with one-to-one correspondence, and which guides forward movement while pressing the spring S during forward movement of the needle bundle 123, are formed in an inner side surface of the opening 120b2 in the rear surface of the needle bundle cover 120b, and during rearward movement of the conveyor 125, a restoring force of the spring S causes the plurality of needles 123c and 123c' protruding through the plurality of needle protrusion holes 120b1 of the needle bundle cover 120b to return to an inner portion of the needle bundle cover 120b.

A pair of locking protrusions 120b4 facing each other are formed on an inner side surface close to an edge of the opening 120b2 in the rear surface of the needle bundle cover 120b.

The pair of locking protrusions 120b4 rotate after being inserted into an L-shaped locking groove 120a2 formed in an outer circumferential surface of a tip of the end cover 120a1 of the handpiece housing 120a and fix the needle bundle cover 120b in a state in which the needle bundle cover 120b is locked to the end cover 120a1 of the handpiece housing 120a, and a catching groove 120a2' that prevents the locked state from being released is formed in the L-shaped locking groove 120a2.

The skin beautifying device 100 according to the present disclosure that is configured as described above operates as follows.

When an operator of the skin beautifying device 100 fixes the needle bundle cover 120b in a state in which the needle bundle cover 120b is locked to the end cover 120a1 of the handpiece housing 120a, the pair of needle bundle detection parts 128b of the needle bundle detection plate 128 and the pair of needle bundle detection contact point parts 129b electrically come into contact, and simultaneously, the needle bundle detection switch 128c is turned on due to a magnetic force of the magnetic part 129c.

In addition, the memory 129d disposed on the needle bundle cover plate 129 records the number of contacts every time the pair of needle bundle detection parts 128b and the pair of needle bundle detection contact point parts 129b electrically come into contact, and the number of contacts recorded in the memory 129d is transmited to the controller 111.

In the above-described state in which the needle bundle cover 120b is fixed while locked to the end cover 120a1 of the handpiece housing 120a, and the needle bundle detection switch 128c is turned on, when the operator of the skin beautifying device 100 inputs a motor driving request by pressing the button 121a of the handpiece housing 120a, and the step motor driver 121 transmits the motor driving request to the controller 111, the controller 111 outputs the motor driving signal and transmits the motor driving signal to the step motor driver 121.

Accordingly, the step motor driver 121 that has received the motor driving signal repeatedly performs, at predetermined time intervals, a skin beautifying process of outputting a forward movement signal and then outputting a rearward movement signal after an elapse of the AC application time.

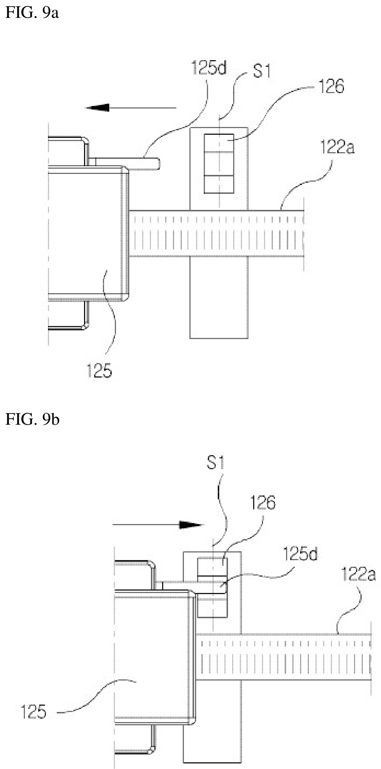

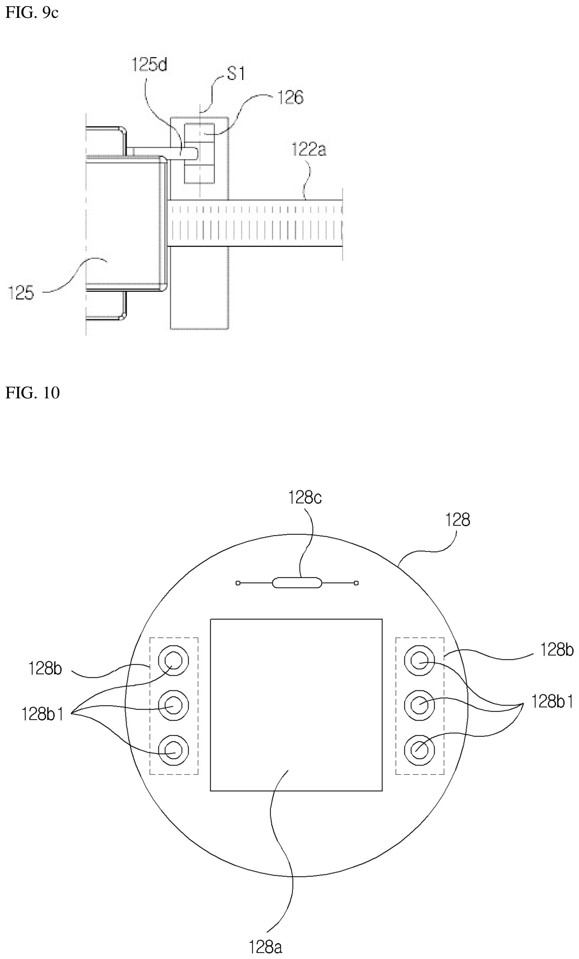

While the step motor driver 121 performs the skin beautifying process as described above, as shown in FIG. 9, the conveyor 125 performs a skin beautifying process of moving forward a moving distance corresponding to the forward movement signal of the step motor 122 (see (a) of FIG. 9), pushing the needle bundle 123 so that the plurality of needles 123c and 123c' fixed to the needle bundle 123 are precisely inserted into the skin to an extent corresponding to the forward movement signal, supplying the AC to the plurality of needles 123c and 123c' of the needle bundle 123 via the pair of terminal pins 125a1, the pair of AC supply terminal parts 124a of the AC terminal plate 124, and the positive electrode terminal part 123a2' and the negative electrode terminal part 123a3' of the needle bundle 123 so that the AC is conducted into the skin through the plurality of needles 123c and 123c' for a predetermined amount of time and local thermal damage is applied to the skin to form fine wounds for promoting regeneration of the skin, and moving rearward a moving distance corresponding to the rearward movement signal of the step motor 122 (see (b) of FIG. 9), and returning to the starting point position (the position of a detection line S1 marked in FIG. 9).

At this time, in a preferable embodiment of the present disclosure, by setting the motor driving signal of the controller 111 so that the ball screw rotating shaft 122a moves 1 mm forward or rearward per 1 turn of the step motor 122, and the ball screw rotating shaft 122a moves 0.0025 mm forward or rearward per 1 pulse of the forward movement signal or the rearward movement signal, it is possible to precisely adjust a degree of insertion of the plurality of needles 123c and 123c' fixed to the needle bundle 123 in consideration of skin thickness (for example, a thickness from epidermis to dermis of the skin) to an extent that a sufficient skin beautification effect is expected.

In addition, in the skin beautifying process of the conveyor 125, as the operator of the skin beautifying device 100 stops pressing the button 121a of the handpiece housing 120a and stops inputting the motor driving request, the step motor driver 121 stops transmitting the motor driving request to the controller 111, and accordingly, the controller 111 stops outputting the motor driving signal, and the output of the motor driving signal is stopped without being performed further.

When an error occurs in the degree of insertion of the needles 123c and 123c' due to forward or rearward movement of the conveyor 125 that is caused by a mechanical rotating operation of the ball screw rotating shaft 122a of the step motor 122 being repeatedly performed in a state in which the skin beautifying process of the conveyor 125 is stopped after being repeatedly performed at predetermined tim intervals, the error in the degree of insertion of the needles 123c and 123c' can be easily corrected by correcting a starting point position of the conveyor 125 using the starting point position detector 126.

In this case, when a starting point correction request is input by the operator of the skin beautifying device 100 manipulating a button (not illustrated), the controller 111 outputs a starting point correction driving signal to the step motor driver 121 so that the step motor driver 121 outputs a starting point correction signal, and when deviation of the conveyor 125 from the starting point position is detected by the starting point position detector 126 while the step motor 122 rotates the ball screw rotating shaft 122a forward a predetermined number of forward rotations corresponding to the starting point correction signal as shown in (c) of FIG. 9, for example, when it is detected that the position detection protrusion 125d of the conveyor 125 has deviated from the detection line S1 as shown in (c) of FIG. 9, the controller 111 may stop outputting the starting point correction driving signal to the step motor driver 121 and may corrects the current position of the conveyor 125, for example, the position thereof right after the position detection protrusion 125d of the conveyor 125 has deviated from the detection line S1 as shown in (c) of FIG. 9, to the starting point position.

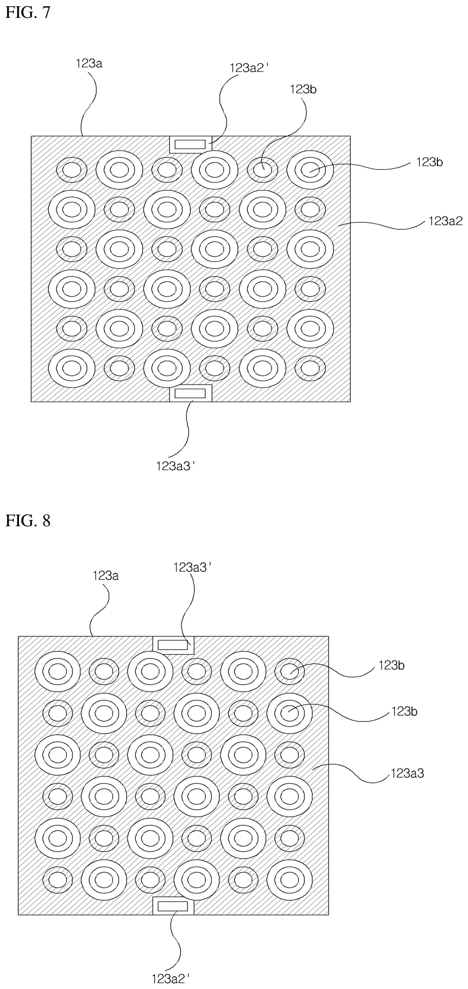

Meanwhile, in an embodiment of the present disclosure, while the skin beautifying process of the conveyor 125 is performed, since all of the positive polarity needles 123c among the plurality of needles 123c and 123c' fixed to the needle bundle 123 are connected to be electrically connected to the positive electrode thin film conductive layer 123a2 as shown in FIG. 6, and all of the negative polarity needles 123c' are connected to be electrically connected to the negative electrode thin film conductive layer 123a3 as shown in FIG. 7, the positive polarity and the negative polarity are alternately formed in the plurality of needles 123c and 123c' disposed in a lattice shape, and AC can be evenly and stably supplied.

On the other hand, in an embodiment of the present disclosure, while the skin beautifying process of the conveyor 125 is performed, every time a process in which the needle bundle cover 120b is separated from the end cover 120a1 of the handpiece housing 120a and then coupled thereto again is repeated, the number of times the pair of needle bundle detection parts 128b and the pair of needle bundle detection contact point parts 129b electrically come into contact is recorded in the memory 129d and transmited to the controller 111.

In this case, when the number of contacts recorded in the memory 129d of the needle bundle cover plate 129 that is accommodated in the needle bundle cover 120b exceeds a predetermined value or more, the controller 111 generates an alert for stopping use of a needle bundle 123 that is used a predetermined number of times or more and requests for replacement of the needle bundle 123, and thus safe use of the needle bundle 123 can be expected.

According to the present disclosure, it is possible to precisely adjust a degree of insertion of a plurality of needles fixed to a needle bundle in consideration of skin thickness (for example, a thickness from epidermis to dermis of the skin) to an extent that a sufficient skin beautification effect is expected, and when an error occurs in the degree of insertion of the needles due to forward or rearward movement of a conveyor during use, the error in the degree of insertion of the needles can be easily corrected by correcting a starting point position of the conveyor using a starting point position detector.

According to the present disclosure, since all positive polarity needles among the plurality of needles fixed to the needle bundle are connected to be electrically connected to a positive electrode thin film conductive layer, and all negative polarity needles are connected to be electrically connected to a negative electrode thin film conductive layer, the positive polarity and the negative polarity are alternately formed in the plurality of needles disposed in a lattice shape, and AC can be evenly and stably supplied.

According to the present disclosure, by generating an alert for stopping use of a needle bundle that is used a predetermined number of times or more and requesting for replacement of the needle bundle, safe use of the needle bundle can be expected.

## Claims

1. A skin beautifying device, which is a skin beautifying device (100) that comprises a main body (110) and a handpiece (120) connected to the main body (110) via a wire and forms fine wounds for promoting regeneration of skin by a plurality of needles (123c, 123c') installed in the handpiece (120) being inserted into the skin, conducting alternating current (AC) for a predetermined amount of time, and applying local thermal damage,
wherein the main body (110) is configured to include:
a controller (111) that adjusts an AC application time during which AC is applied to the plurality of needles (123c, 123c'), that repeatedly performs, at predetermined time intervals, a process of outputting a motor driving signal for adjusting a degree of insertion of the plurality of needles (123c, 123c') into the skin while a motor driving request is present, supplying the AC to the plurality of needles (123c, 123c') inserted into the skin during the AC application time, and then taking the plurality of needles (123c, 123c') out of the skin, and that stops outputting the motor driving signal in response to a stop of the motor driving request;
a power adjuster (112) that adjusts a voltage level of direct current (DC) power that is supplied to generate AC supplied to the handpiece (120); and
an AC generator (113) that converts the DC power supplied from the power adjuster (112) into AC power to generate the AC supplied to the handpiece (120), and
the handpiece (120) is configured to include:
a handpiece housing (120a) that is hollow and serves as a handle;
a needle bundle cover (120b) that is hollow, is coupled to or separated from an end of the handpiece housing (120a), and has a plurality of needle protrusion holes (120b1), which correspond one-to-one to the plurality of needles (123c, 123c'), formed in a front surface and an opening (120b2) formed at a rear surface;
a step motor driver (121) that is accommodated in the handpiece housing (120a), that repeatedly performs, at predetermined time intervals, a skin beautifying process of transmitting the motor driving request input by manipulation of a button (121a) to the controller (111), outputting a forward movement signal upon receiving the motor driving signal, and then outputting a rearward movement signal after an elapse of the AC application time, and that stops transmitting the motor driving request to the controller (111) in response to a stop of an input of the motor driving request;
a step motor (122) that is accommodated in the handpiece housing (120a) and has a ball screw rotating shaft (122a) that performs a predetermined number of forward rotations corresponding to the forward movement signal or performs a predetermined number of rearward rotations corresponding to the rearward movement signal;
a needle bundle (123) that is accommodated in the needle bundle cover (120b) and in which a positive electrode thin film conductive layer (123a2) and a negative electrode thin film conductive layer (123a3) are stacked on a front surface and a rear surface, respectively, of a central insulating plate (123a1), a surface of the positive electrode thin film conductive layer (123a2) and a surface of the negative electrode thin film conductive layer (123a3) pass through a front surface and a rear surface, respectively, of a needle bundle plate (123a) covered with an insulating layer (123a4), the plurality of needles (123c, 123c'), which correspond one-to-one to a plurality of through-holes (123b) arranged in a lattice shape, are fixed by soldering while inserted into the plurality of through-holes (123b), during supply of the AC, the plurality of needles (123c, 123c') alternately have a positive polarity and a negative polarity while neighboring each other in a horizontal direction and a vertical direction of the lattice shape, all of the positive polarity needles (123c) among the plurality of needles (123c, 123c') are connected to be electrically connected to the positive electrode thin film conductive layer (123a2) formed on the front surface, all of the negative polarity needles (123c') among the plurality of needles (123c, 123c') are connected to be electrically connected to the negative electrode thin film conductive layer (123a3) formed on the rear surface, and a positive electrode terminal part (123a2') connected to the positive electrode thin film conductive layer (123a2) and a negative electrode terminal part (123a3') connected to the negative electrode thin film conductive layer (123a3) are formed on a left side edge and a right side edge, respectively, of the front surface;
an AC terminal plate (124) that is accommodated in the needle bundle cover (120b) and has a front surface adhered and coupled to a rear surface of the needle bundle (123) and a pair of AC supply terminal parts (124a), which are spaced apart from each other and connected to be electrically connected to the positive electrode terminal part (123a2') and the negative electrode terminal part (123a3') of the needle bundle (123), disposed on a surface of a central portion of a rear surface; and
a conveyor (125) that is accommodated in the handpiece housing (120a), has a pair of terminal pins (125a1), which come into contact with the pair of AC supply terminal parts (124a) of the AC terminal plate (124) and supply the AC to the plurality of needles (123c, 123c') of the needle bundle (123), protruding from a front end protruding part (125a), is connected to be electrically connected to the pair of terminal pins (125a1) through wires inserted into wire connection holes (125b1) formed in a pair of protruding parts (125b) of left and right side surface parts, applies the AC supplied from the AC generator (113) to the pair of terminal pins (125a1), is screw-coupled to the ball screw rotating shaft (122a) of the step motor (122) through a screw hole (125c) in a rear surface part, moves forward from a starting point position and then moves rearward and returns to the starting point position, moves forward a moving distance corresponding to a forward movement signal of the step motor (122) and pushes the needle bundle (123) so that the plurality of needles (123c, 123c') fixed to the needle bundle (123) are precisely inserted into the skin to an extent corresponding to the forward movement signal, supplies the AC to the plurality of needles (123c, 123c') of the needle bundle (123) via the pair of terminal pins (125a1), the pair of AC supply terminal parts (124a) of the AC terminal plate (124), and the positive electrode terminal part (123a2') and the negative electrode terminal part (123a3') of the needle bundle (123) so that the AC is conducted into the skin through the plurality of needles (123c, 123c') for a predetermined amount of time and local thermal damage is applied to the skin to form fine wounds for promoting regeneration of the skin, and moves rearward a moving distance corresponding to a rearward movement signal of the step motor (122) and returns to the starting point position.

2. The skin beautifying device of claim 1, further comprising a starting point position detector (126) that is accommodated in the handpiece housing (120a) and outputs a starting point position detection signal and transmits the starting point position detection signal to the controller (111) upon detecting that the conveyor (125) has moved rearward the moving distance corresponding to the rearward movement signal of the step motor (122) and a position detection protrusion (125d) protruding from a rear side portion of the conveyor (125) has returned to the starting point position,
wherein, when a starting point correction request is input by manipulation of a button (not illustrated), the controller (111) outputs a starting point correction driving signal to the step motor driver (121) so that the step motor driver (121) outputs a starting point correction signal, and when deviation of the position detection protrusion (125d) of the conveyor (125) from the starting point position is detected by the starting point position detector (126) while the step motor (122) rotates the ball screw rotating shaft (122a) forward a predetermined number of forward rotations corresponding to the starting point correction signal, the controller (111) stops outputting the starting point correction driving signal to the step motor driver (121) and corrects the current position of the conveyor (125) to the starting point position.

3. The skin beautifying device of claim 1, further comprising a needle bundle holder (127) that is accommodated in the needle bundle cover (120b), allows the AC terminal plate (124) to be fixed as an edge portion of the needle bundle (123), which is adhered and coupled to a rear surface, is caught at an opening in a front surface of a body (127a) having a shape that is open in a front-rear direction, has a terminal pin hole (127b1), through which the pair of terminal pins (125a1) of the conveyor (125) pass and come into contact with the pair of AC supply terminal parts (124a) of the AC terminal plate (124), formed in a central portion of a rear surface cover (127b) that covers an opening in a rear surface of the body (127a), has a plurality of protrusions (127b2) formed along an edge of the rear surface cover (127b), and allows the needle bundle (123) to move forward when a front end of the conveyor (125) moves forward and pushes the rear surface cover (127b) while in contact with the rear surface cover (127b).

4. The skin beautifying device of claim 1, further comprising:
a needle bundle detection plate (128) that is accommodated in the handpiece housing (120a), has a central through-hole (128a) formed therein through which the front end protruding part (125a) of the conveyor (125) passes, has a front surface at which the pair of protruding parts (125b) of the left and right side surface parts of the conveyor (125) are caught and fixed, has a pair of needle bundle detection parts (128b), which are spaced apart from each other with the central through-hole (128a) disposed therebetween and include at least one or more detection pins (128b1), disposed side by side on the front surface, and has a needle bundle detection switch (128c) disposed at a proper place on the front surface that is spaced apart from the pair of needle bundle detection parts (128b); and
a needle bundle cover plate (129) that is accommodated in the needle bundle cover (120b), has a central through-hole (129a) formed therein through which the front end protruding part (125a) of the conveyor (125) passes, has a pair of needle bundle detection contact point parts (129b), which are spaced apart from each other with the central through-hole (129a) disposed therebetween and include at least one or more detection contact points (129b1), disposed side by side on a rear surface facing the needle bundle detection plate (128), and has a magnetic part (129c), which turns on the needle bundle detection switch (128) due to a magnetic force when the pair of needle bundle detection parts (128b) of the needle bundle detection plate (128) and the pair of needle bundle detection contact point parts (129b) electrically come into contact as the needle bundle cover (120b) is fixed while locked to an end cover (120a1) of the handpiece housing (120a), and a memory (129d), which records a number of contacts every time the pair of needle bundle detection parts (128b) and the pair of needle bundle detection contact point parts (129b) electrically come into contact, disposed at proper places on the rear surface.

5. The skin beautifying device of claim 1, wherein the controller (111) generates an alert for stopping use of the needle bundle (123) when the number of contacts recorded in the memory (129d) of the needle bundle cover plate (129) that is accommodated in the needle bundle cover (120b) exceeds a predetermined value or more.

6. The skin beautifying device of claim 1, wherein the step motor driver (121) transmits the motor driving request input by manipulation of a button (121a) to the controller (111) in a state in which the needle bundle detection switch (128c) of the needle bundle detection plate (128) accommodated in the handpiece housing (120a) is turned on.

7. The skin beautifying device of claim 1, wherein a plurality of movement guide grooves (120b3), into which a spring (S) is inserted, into which the plurality of protrusions (127b2) formed along the edge of the rear surface cover (127b) of the needle bundle holder (127) are inserted with one-to-one correspondence, and which guides forward movement while pressing the spring (S) during forward movement of the needle bundle (123), are formed in an inner side surface of an opening (120b2) in the rear surface of the needle bundle cover (120b), and during rearward movement of the conveyor (125), a restoring force of the spring (S) causes the plurality of needles (123c, 123c') protruding through the plurality of needle protrusion holes (120b1) of the needle bundle cover (120b) to return to an inner portion of the needle bundle cover (120b).

8. The skin beautifying device of claim 1, wherein a pair of locking protrusions (120b4) facing each other are formed on an inner side surface close to an edge of the opening (120b2) in the rear surface of the needle bundle cover (120b), the pair of locking protrusions (120b4) rotate after being inserted into an L-shaped locking groove (120a2) formed in an outer circumferential surface of a tip of the end cover (120a1) of the handpiece housing (120a) and fix the needle bundle cover (120b) in a state in which the needle bundle cover (120b) is locked to the end cover (120a1) of the handpiece housing (120a), and a catching groove (120a2') that prevents the locked state from being released is formed in the L-shaped locking groove (120a2).
